(19) Europäisches Patentamt
European Patent Office
Office européen des brevets

(11) Veröffentlichungsnummer : **0 202 196**
**B1**

(12) # EUROPÄISCHE PATENTSCHRIFT

(45) Veröffentlichungstag der Patentschrift : **15.03.89**

(51) Int. Cl.⁴ : **C 07 D317/30**, C 07 D319/06, G 03 F 7/10, G 03 F 7/08

(21) Anmeldenummer : **86810207.0**

(22) Anmeldetag : **06.05.86**

(54) **Cyclische Acetale oder Ketale von beta-Ketoestern oder -amiden.**

(30) Priorität : **15.05.85 CH 2089/85**

(43) Veröffentlichungstag der Anmeldung : **20.11.86 Patentblatt 86/47**

(45) Bekanntmachung des Hinweises auf die Patenterteilung : **15.03.89 Patentblatt 89/11**

(84) Benannte Vertragsstaaten : **BE CH DE FR GB IT LI NL SE**

(56) Entgegenhaltungen :
**CHEMICAL ABSTRACTS, Band 87, 1977, Seite 551, Zusammenfassung Nr. 68333n, Columbus, Ohio, US; & JP-A-76 127 078 (NIPPON CHEMICAL INDUSTRIAL CO., LTD) 05-11-1976**

(73) Patentinhaber : **CIBA-GEIGY AG**
**Klybeckstrasse 141**
**CH-4002 Basel (CH)**

(72) Erfinder : **Roth, Martin, Dr.**
**Oberdorf**
**CH-1711 Giffers (CH)**

**EP 0 202 196 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft cyclische Acetale oder Ketale von β-Ketoestern oder -amiden, Zusammensetzungen enthaltend diese Verbindungen in Kombination mit photolytisch-induziert säureabgebenden Verbindungen, ein Verfahren zur Erzeugung von positiven Abbildungen unter Verwendung besagter Zusammensetzungen, sowie die Verwendung der Zusammensetzungen zur Herstellung von Druckplatten, gedruckten Schaltungen, integrierten Schaltungen oder silberlosen photographischen Filmen.

Positiv arbeitende Photoresistsysteme sind bekannt. Ein Typ solcher Lake besteht aus einer photoaktivierbaren säureabspaltenden Verbindung A, einer weiteren Verbindung B, die sich unter dem Einfluss einer Säure chemisch umsetzt und gegebenenfalls einem Bindemittel.

Zur Herstellung von Abbildungen wird ein Substrat mit dem Lacksystem beschichtet und in einem anschliessenden Arbeitsgang an ausgewählten Stellen bestrahlt. Dabei zersetzt sich die Verbindung A an den der Strahlung ausgesetzten Stellen und dort entsteht eine Protonen- oder Lewissäure. Diese Säure wiederum induziert die chemische Umsetzung der Verbindung B. Die Verbindung B wirkt als « Lösungsinhibitor » für das Bindemittel bzw. sie ist in geeigneten Lösungsmitteln (Entwickler) nicht oder nur schlecht löslich. Nach ihrem Abbau geht diese Eigenschaft verloren und die bestrahlten Stellen lassen sich unter Einwirkung eines Entwicklers ablösen, während die unbestrahlten Stellen der Schicht erhalten bleiben.

Positiv arbeitende Photolacksysteme, die nach diesem Prinzip arbeiten, sind beispielsweise in der DE-AS 2,718, 254 oder in der US-PS 3,779,778 beschrieben. Bei den dort verwendeten « Lösungsinhibitoren » handelt es sich um spezielle Acetale oder Ketale.

Abbildungssysteme der oben beschriebenen Art besitzen eine Vielzahl von Parametern, die die Qualität des entstehenden Bildes beeinflussen können. Dazu zählen beispielsweise : spektrale Empfindlichkeit des Lacksystems oder Quantenausbeute der Photoreaktion (Anzahl der umgewandelten Lösungsinhibitoren/absorbiertem Strahlungsquant), Haftung der unbestrahlten Schicht auf dem Substrat oder Löslichkeitsunterschiede zwischen bestrahlten und unbestrahlten Stellen der Schicht gegenüber dem Entwickler.

Es besteht insbesondere ein Bedarf nach Verbindungen, die sich in Positiv-Photolacken einsetzen lassen, und die die Herstellung von einerseits sehr dünnen Lackschichten im Bereich von 0,5 µm bis 10 µm und andererseits sehr dicken Lackschichten bis zu 100 µm Dicke gestatten. Ferner besteht ein Bedarf nach Systemen, mit denen sich möglichst grosse Löslichkeitsunterschiede zwischen bestrahlten und unbestrahlen Stellen der Schicht erreichen lassen.

Die vorliegende Erfindung betrifft Verbindungen der Formel I

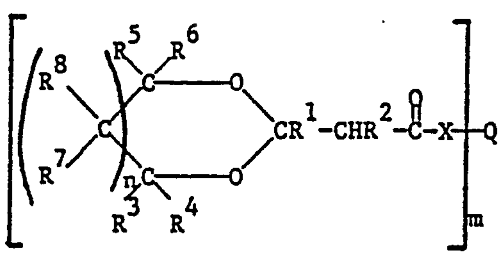

(I)

worin $R^1$ und $R^2$ unabhängig voneinander Wasserstoff, $C_1$-$C_8$Alkyl, Phenyl, Naphthyl, $C_5$-$C_7$Cycloalkyl, $C_7$-$C_{14}$Aralkyl oder $C_7$-$C_{14}$Alkaryl bedeuten, wobei besagte organische Reste gegebenenfalls durch ein bis drei Halogenatome, Hydroxy-, Alkoxy-, Nitro-, Cyan- oder Aminogruppen substituiert sind, worin $R^3$, $R^4$, $R^5$, $R^6$, $R^7$ und $R^8$ unabhängig voneinander Wasserstoff oder $C_1$-$C_4$Alkyl bedeuten, worin X —O— oder —$NR^9$— ist, worin $R^9$ Wasserstoff oder $C_1$-$C_4$Alkyl ist, n 0 oder 1 bedeutet, m 2, 3 oder 4 ist, und worin Q einen m-wertigen aliphatischen, cycloaliphatischen, aromatischen, araliphatischen oder fünf- oder sechsgliedrigen heterocyclischen Rest darstellt, wobei beim Vorliegen mehrerer Ester- oder Amidreste im Molekül, die Gruppen $R^1$ bis $R^9$ innerhalb der oben gegebenen Definitionen unterschiedlich sein können, und wobei im Falle n = 0 die beiden Kohlenstoffatome direkt miteinander verbunden sind, so dass ein fünfgliedriger heterocyclischer Rest entsteht.

Bei $R^1$ und $R^2$ als $C_1$-$C_8$Alkyl handelt es sich um geradkettige oder verzweigtkettige Reste. Beispiele für Gruppen dieser Art sind Methyl, Ethyl, n-Propyl, Isopropyl, n-Butyl, sek.-Butyl, n-Pentyl, Isoamyl, n-Hexyl oder n-Octyl. Bevorzugt werden geradkettige Reste und ganz besonders Methyl. $R^1$ bedeutet vorzugsweise auch Phenyl und Wasserstoff. $R^2$ ist vorzugsweise Wasserstoff.

Die Reste $R^3$ bis $R^8$ bedeuten als $C_1$-$C_4$Alkyl beispielsweise Methyl, Ethyl, n-Propyl, Isopropyl oder n-Butyl. Bevorzugt wird Methyl. $R^3$ bis $R^8$ sind vorzugsweise Wasserstoff.

Handelt es sich bei $R^1$ oder $R^2$ um $C_5$-$C_7$Cycloalkyl, so sind dies beispielsweise Cyclopentyl, Cyclohexyl oder Cycloheptyl, besonders bevorzugt jedoch Cyclohexyl.

$R^1$ und $R^2$ bedeuten als $C_7$-$C_{14}$Aralkyl beispielsweise Benzyl, α-Methylbenzyl, α,α-Dimethylbenzyl oder 2-Phenylethyl. Bevorzugt ist Benzyl.

Als $C_7$-$C_{14}$Alkaryl bedeuten $R^1$ und $R^2$ beispielsweise o-, m- oder p-Tolyl, Xylyl oder Mesityl. Bevorzugt wird Tolyl.

Stellen $R^1$ und $R^2$ durch ein bis drei Halogenatome, Alkoxy-, Nitro-, Cyan- oder Aminogruppen substituierte organische Reste dar, so handelt es sich dabei beispielsweise um 2-Hydroxyethyl, 2- oder 3-Hydroxypropyl, 2-, 3- oder 4-Hydroxyphenyl, 2-Chlor- oder 2-Bromethyl, 2-, 3- oder 4-Chlorphenyl, 2-, 3- oder 4-Chlorbenzyl, 2-, 3- oder 4-Nitrophenyl, 2-, 3- oder 4-Cyanphenyl, sowie um 2-, 3- oder 4-Aminophenyl.

Q ist definitionsgemäss ein zwei-, drei- der vierwertiger organischer Rest der oben angegebenen Art. Zu den zweiwertigen Resten Q zählen beispielsweise geradkettige oder verzweigkettige Alkylenreste, die gegebenenfalls durch Heteroatome, wie —O— oder —S—, in der Kette unterbrochen sein können.

Bevorzugt werden geradkettige oder verzweigkettige $C_2$-$C_{18}$Alkylenreste $—C_nH_{2n}—$ (n = 2-18), und ganz besonders geradkettige $C_2$-$C_{12}$Alkylenreste. Beispiele für Gruppen dieses Typs sind Ethylen, Trimethylen, Tetramethylen, Pentamethylen, Hexamethylen, Heptamethylen, Octamethylen, Nonamethylen, Decamethylen, Undecamethylen oder Dodecamethylen.

Als zweiwertige Reste Q kommen aber auch substituierte oder unsubstituierte Cycloalkylengruppe in Frage. Bei den alkylsubstituierten Cycloalkylenresten können die Alkylgruppen dabei einen Teil der Kette bilden, wie beispielsweise in $—CH_2—C_6H_{10}—CH_2—$, oder die Substituenten befinden sich in der Position eines Wasserstoffatoms, wie beispielsweise in $—(CH_3)C_6H_8(CH)—$. Bevorzugt sind Reste, die sich von Cyclohexylen ableiten, also 1,2- 1,3- oder 1,4-Cyclohexylen oder auch Derivate von Bis-(hydroxymethyl)-cyclohexan nach dem Entfernen beider Hydroxygruppen. Insbesondere bevorzugt sind die entsprechenden 1,4-Cyclohexylenderivate.

Als zweiwertige Reste Q sind aber auch substituierte oder unsubstituierte monocyclische Arylenreste denkbar. Beispiele dafür sind o-, m- oder p-Phenylen oder Verbindungen, die sich von Bis-(hydroxymethyl)-phenylderivaten ableiten, nach Entfernung der beiden Hydroxygruppen. Bevorzugte Vertreter dieser letzten Gruppe sind entsprechende 1,3- oder 1,4-Derivate.

Zu den zweiwertigen aromatischen Reste Q zählen aber auch Verbindungen enthaltend mehrere aromatische Reste, entweder kondensiert oder auch linear anelliert. Dazu zählen insbesondere Verbindungen der Formel

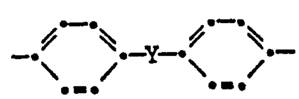

worin Y $—CH_2—$, $—C(CH_3)_2—$, —O—, —S—, $—SO_2—$ oder —CO— bedeutet.

Zweiwertige heterocyclische Reste Q leiten sich beispielsweise von Furan, Pyran, Thiophen oder s-Triazin ab.

Stellt Q einen dreiwertigen organischen Rest dar, so handelt es sich beispielsweise um einen dreiwertigen gesättigten aliphatischen Rest der allgemeinen Formel $C_nH_{2n-1}$. Solche Reste umfassen vorzugsweise drei bis neun Kohlenstoffatome. Die Bindung zum restlichen Molekül der Formel I erfolgt über unterschiedliche Kohlenstoffatome. Bevorzugte Reste leiten sich von Glycerin oder von Trimethylolpropan ab. Dreiwertige Reste Q können aber auch cycloaliphatische oder aromatische Gruppen aufweisen. Bevorzugte Reste dieses Typs leiten von 1,3,5-Trimethylolbenzol ab bzw. vom entsprechenden hydrierten Cyclohexanderivat.

Stellt Q einen vierwertigen organischen Rest dar, so handelt es sich beispielsweise um einen vierwertigen aliphatischen gesättigten Rest der allgemeinen Formel $C_nH_{2n-2}$. Solche Reste umfassen vorzugsweise vier bis neun Kohlenstoffatome. Ein bevorzugter Vertreter dieser Gruppe leitet sich von Pentaerithrit ab. Die Bindung zum restlichen Molekül der Formel I erfolgt über unterschiedliche Kohlenstoffatome. Vierwertige Reste Q können aber auch aromatische oder cycloaliphatische Gruppen aufweisen. Bevorzugte Reste diese Typs leiten sich von 1,2,4,5-Tetramethylolbenzol ab, oder auch von 3,3', 4,4'-Tetramethylolbenzophenon bzw. den entsprechenden 1,1'2,2'-Derivaten davon. Vierwertige Reste Q können sich aber auch von den entsprechenden hydrierten cycloaliphatischen Derivaten der oben erwähnten aromatischen Tetramethylolverbindungen ableiten.

Bevorzugt werden Verbindungen der Formel I, worin $R^1$ und $R^2$ Wasserstoff, $C_1$-$C_4$Alkyl oder Phenyl sind, $R^3$, $R^4$, $R^5$ und $R^6$ unabhängig voneinander Wasserstoff oder Methyl bedeuten, Y —O— oder —NH— darstellt, n 0 bedeutet, m 2 oder 3 ist, und worin Q ein m-wertiger aliphatischer, cycloaliphatischer, oder araliphatischer Rest ist.

Ganz besonders bevorzugt werden Verbindungen der Formel I, worin $R^1$ und $R^2$ unabhängig voneinander Wasserstoff, Methyl oder Phenyl sind, $R^3$, $R^4$, $R^5$ und $R^6$ Wasserstoff bedeuten, X —O— oder —NH— ist, n 0 darstellt, m 2 ist, und worin Q ein zweiwertiger Rest ist, der ausgewählt wird aus der Gruppe bestehend aus $C_2$-$C_{18}$Alkylen, Xylylen, oder Hexahydroxylylen.

Ganz besonderes Interesse finden Verbindungen der Formel I, worin $R^1$ und $R^2$ unabhängig voneinander Wasserstoff, Methyl oder Phenyl bedeuten, $R^3$, $R^4$, $R^5$ und $R^6$ Wasserstoff darstellen, X —O— oder —NH— ist, n 0 bedeutet, m 3 ist, und worin Q sich ableitet von Trimethylolpropan oder Glyzerin nach Entfernung der Alkoholgruppen. Ebenfalls Beachtung finden Verbindungen der Formel I,

worin $R^1$ und $R^2$ unabhängig voneinander Wasserstoff, Methyl oder Phenyl bedeuten, $R^3$, $R^4$, $R^5$ und $R^6$ Wasserstoff darstellen, X —O— oder —NH— ist, n 0 ist, m 4 bedeutet, und worin Q sich ableitet von Penthaerithrit nach Entfernung der Alkoholgruppen.

Ganz besonders bevorzugt werden Verbindungen der Formel I, worin n 0 bedeutet. Ebenfalls bevorzugt werden Verbindungen der Formel I, worin m 2 oder 3 ist. Ganz besondere Beachtung finden Verbindungen der Formel I, worin $R^1$ Methyl ist und die Reste $R^2$ bis $R^8$ Wasserstoff darstellen.

Besonders bevorzugt werden Ester der Formel I, also Verbindungen mit X = —O—.

Die Herstellung der erfindungsgemässen β-Acetalester oder -amide der Formel I erfolgt nach an sich bekannten Verfahren.

Bei einer Herstellungsvariante geht man von einem β-Ketoester oder -amid der Formel II aus und setzt diesen (dieses) mit einem Diol der Formel III zum entsprechenden Acetal bzw. Ketal gemäss dem folgenden Schema 1 um.

Schema 1

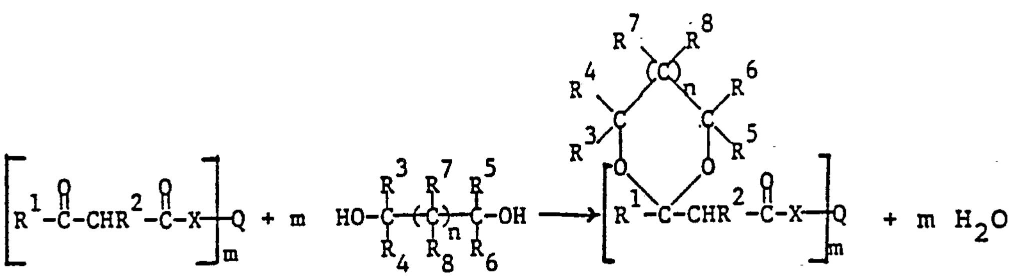

(II) (III) (I)

Dabei haben die Gruppen $R^1$ bis $R^8$, X und Q, sowie die Indizes m und n, die oben definierte Bedeutung.

Die Reaktion erfolgt in Gegenwart eines sauren Katalysators. Beispiele für solche Katalysatoren sind anorganische Säuren, wie Salzsäure, Schwefelsäure, Salpetersäure oder Phosphorsäure, oder auch organische Säuren, wie z. B. p-Toluolsulfonsäure. Die Reaktion kann in An- oder in Abwesenheit eines Lösungsmittels durchgeführt werden. Lösungsmittel sind in der Regel organischer Natur und inert. Beispiele dafür sind halogenierte Kohlenwassertoffe, wie Methylenchlorid, Chloroform, Tetrachlorkohlenstoff, Dichlorethan oder Chlorbenzol, sowie aromatische Lösungsmittel, wie Benzol, Toluol oder Xylol. Die Auswahl eines Lösungsmittels für eine bestimmte Reaktionrichtet sich nach den jeweils einzusetzenden Reaktionspartnern, und kann vom Fachmann anhand von Routineversuchen ermittelt werden.

Die β-Ketoester oder -amide der Formel II und die Diole der Formel III sind an sich bekannte Verbindungen. Sie sind teilweise im Handel erhältlich oder können zumindest nach Standardreaktionen der organischen Chemie hergestellt werden. Verbindungen der Formel II können z. B. durch Umsetzung der entsprechenden Alkohole oder Amine mit Diketen erhalten werden. Eine weitere Möglichkeit zur Herstellung der Verbindungen der Formel I besteht in der Umesterung eines β Acetal- oder Ketalesters der Formel IV mit dem mehrwertigen Alkohol der Formel V bzw. in der Amidbildung durch Umsetzung des Esters der Formel IV mit einem mehrwertigen Amin der Formel V, wie dies im nachfolgenden Schema 2 angegeben ist.

Schema 2

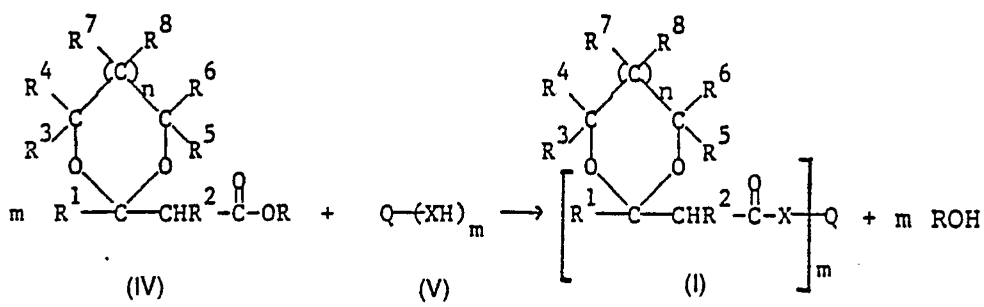

(IV) (V) (I)

Dabei haben die Reste $R^1$-$R^8$, X und Q, sowie die Indizes m und n, die weiter oben definierte Bedeutung. R ist ein einwertiger Kohlenwasserstoffrest, bevorzugt $C_1$-$C_4$Alkyl, und besonders bevorzugt Methyl oder Ethyl.

Die Umesterung bzw. Amidbildung nach Schema 2 ist an sich bekannt und wird in der Regel unter Einwirkung eines basischen oder sauren Katalysators durchgeführt, beispielsweise in Gegenwart von

Natronlauge. Die Reaktion kann, wie auch die in Schema 1 beschriebene Acetalbildung, in An- oder Abwesenheit von Lösungsmitteln durchgeführt werden. Die Ausgangsstoffe der Formel V sind an sich bekannte Verbindungen und teilweise im Handel erhältlich. Die Ester der Formel IV können mit der in Schema 1 skizzierten Methode hergestellt werden.

Wie bereits erwähnt, lassen sich die Verbindungen der Formel I zur Herstellung von positiv arbeitenden Photoresistsystemen verwenden.

Die Erfindung betrifft daher auch Zusammensetzungen enthaltend

a) eine Verbindung der Formel I, wie oben definiert, b) eine unter Einwirkung von aktinischer Strahlung eine Säure abspaltende Verbindung, und c) gegebenenfalls ein Bindemittel.

Die Menge an β-Acetal- bzw. -Ketalverbindung der Formel I beträgt in der Regel 5-50 Gew.%, bezogen auf die Gesamtmenge der Zusammensetzung. Bevorzugt verwendet man 10-40 Gew.% der Verbindung der Formel I.

Als strahlungsemfindliche Komponenten, die bei Lichteinwirkung Säure bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenden Kopiermassen verwendet werden, oder auch Halogenverbindungen, die bei Bestrahlung Halogenwasserstoffsäure bilden. Verbindungen dieses Typs sind beispielsweise in den US-PSS 3 515 552, 3 536 489 oder 3 779 778, sowie in den DE-OSS 2 718 259, 2 243 621 oder 2 610 842 beschrieben.

Als strahlungsempfindliche Komponenten b) der erfindungsgemässen Zusammensetzungen eignen sich aber auch kationische Photoinitiatoren aus der Gruppe der Iodonium- oder Sulfoniumsalze. Solche Verbindungen sind beispielsweise in « UV-Curing, Science and Technology » (Editor : S. P. Pappas, Technology Marketing Corp., 642 Westover Road, Stanford, Connecticut, USA) beschrieben.

Insbesondere sind auch Diaryliodosyl-Salze einsetzbar. Solche Verbindungen sind beispielsweise in der EP-A 106 797 beschrieben.

Ferner können Sulfoxoniumsalze als strahlungsempfindliche Verbindungen verwendet werden. Solche Salze sind beispielsweise in der EP-PS 35 969 oder in den EP-A 44 274 bzw. 54 509 beschrieben. Insbesondere zu erwähnen sind aliphatische Sulfoxoniumsalze, die im tiefen UV-Bereich absorbieren, wie beispielsweise in der EP-A 164 314 beschrieben.

Insbesondere lassen sich auch Verbindungen einsetzen, die bei Bestrahlung mit aktinischem Licht Sulfonsäuren freisetzen. Solche Verbindungen sind an sich bekannt und beispielsweise in der GB-A 2 120 263, den EP-A 84 515, 37 152 oder 58 638 bzw. in den US-PSS 4 258 121 oder 4 371 605 beschrieben.

Werden als strahlungsempfindliche säureabspaltende Komponenten b) Salze eingesetzt, so sind diese vorzugsweise in organischen Lösungsmitteln löslich. Besonders bevorzugt handelt es sich bei diesen Salzen um Abscheidungsprodukte mit komplexen Säuren, beispielsweise von Borfluorwasserstoffsäure oder Hexafluorphosphorsäure.

Die Menge der strahlungsempfindlichen Komponente b) der erfindungsgemässen Zusammensetzungen kann je nach Natur und Zusammensetzung des strahlungsempfindlichen Gemisches in weiten Grenzen variiert werden. Günstige Ergebnisse werden erzielt, wenn etwa 0,1 bis 10 Gew.% an Komponente b), bezogen auf den Gesamtfeststoffgehalt, eingesetzt werden. Bei dickeren Schichten über 10 µm empfiehlt es sich, relativ wenig Säurespender zu verwenden. Vorzugsweise setzt man 0,2 bis 5 Gew.% Säurespender, bezogen auf den Gesamtfeststoffgehalt der strahlungsempfindlichen Zusammensetzung, ein.

Den erfindungsgemässen positiv arbeitenden Photoresists können gegebenenfalls auch Binder (c) zugesetzt werden, was besonders zweckmässig ist, wenn es sich bei den lichtempfindlichen Zusammensetzungen um flüssige oder niedrigviskose Gemische handelt. Die Menge des Binders (c) kann 30-90 Gew.%, vorzugsweise 60-90 Gew.% betragen, bezogen auf die gesamte Menge an Komponenten a), b) und c).

Die Wahl des Bindemittels erfolgt je nach dem Anwendungsgebiet und den hierfür geforderten Eigenschaften wie Entwickelbarkeit in wässrigen oder wässrig/organischen Lösungsmittelsystemen oder Adhäsion auf Substraten.

Geeignete Bindemittel c) sind beispielsweise Novolake, die sich von einem Aldehyd, vorzugsweise Acetaldehyd oder Furfuraldehyd, besonders jedoch von Formaldehyd, und einem Phenol ableiten. Die phenolische Komponente dieser Bindemittel ist vorzugsweise Phenol selbst, oder auch halogeniertes Phenol, beispielsweise substituiert mit ein bis zwei Chloratomen, vorzugsweise p-Chlorphenol, oder sie ist ein durch ein bis zwei $C_1$-$C_9$Alkylgruppen substituiertes Phenol, beispielsweise o-, m- oder p-Kresol, ein Xylenol, p-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Ein Teil der phenolischen Hydroxygruppen dieser Novolake kann gegebenenfalls durch Umsetzung mit Chloressigsäure, Isocyanaten, Epoxiden oder Carbonsäureanhydriden modifiziert sein.

Weitere geeignete Bindemittel sind beispielsweise Copolymere von Maleinsäureanhydrid mit Styrol oder Vinylethern oder 1-Alkenen. Ebenfalls als Bindemittel einsetzen lassen sich Copolymere von Estern der Acrylsäure oder der Methacrylsäure mit ethylenisch ungesättigten Säuren, beispielsweise Methacrylsäure oder Acrylsäure.

Vorzugsweise verwendet man als Bindemittel eine alkalilösliche Substanz, beispielsweise einen Novolak (gegebenenfalls modifiziert, wie oben beschrieben, oder copolymere Acrylate und Methacrylate, z. B. Copolymere aus Methylmethacrylat/Ethylacrylat/Methacrylsäure.

Diesen alkalilöslichen Bindemitteln können gegebenenfalls noch weitere Zusatzharze beigegeben werden, wie bei den Positiv-Systemen auf Diazoketon Basis üblich. Zu diesen Zusatzharzen zählen beispielsweise Vinylpolymerisate, wie Polyvinylacetat, Polyacrylate, Poly-(methacrylsäurealkylester) oder Poly-(acrylsäure alkylester), wobei Alkyl = $C_1$-$C_{20}$ ist, Polyvinylether oder Polyvinylpyrrolidone. Im allgemeinen werden jedoch nicht mehr als 20 Gew.%, bezogen auf die Menge an alkalilöslichem Bindemittel, von diesen Zusatzharzen zugefügt.

Die erfindungsgemässe Zusammensetzung kann weitere übliche Zusatzstoffe enthalten, wie z. B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen Zusammensetzungen eignen sich hervorragend als Beschichtungsmittel für Substrate aller Art, z. B. Holz, Textilien, Papier, Keramik, Glas, Kunststoffe, wie Polyester, Polyethylenterephthalat, Polyolefine oder Celluloseacetat, insbesondere in Form von Filmen, sowie von Metallen, wie Al, Cu, Ni, Fe, Zn, Mg oder Co, und von Si oder $SiO_2$, bei denen durch bildmässiges Belichten eine Abbildung aufgebracht werden soll. Ein weiterer Gegenstand vorliegender Erfindung sind die beschichteten Substrate.

Die Erfindung betrifft ferner auch ein Verfahren zur Erzeugung von positiven Abbildungen umfassend folgende Arbeitsschritte :

a) Beschichtung eines Substrates mit einer strahlungsempfindlichen Zusammensetzung wie oben definiert,

b) Belichtung des beschichteten Substrates mit einem vorbestimmten Muster aktinischer Strahlung, und

c) Entwicklung des belichteten Substrates.

Die Herstellung der beschichteten Substrate kann z. B. erfolgen, indem man eine Lösung oder Suspension der Zusammensetzung herstellt.

Die Wahl des Lösungsmittels und die Konzentration richtet sich hauptsächlich nach der Art der Zusammensetzung und nach dem Beschichtungsverfahren. Die Lösung wird mittels bekannten Beschichtungsverfahren auf ein Substrat gleichförmig aufgebracht, z. B. durch Schleudern, Tauchen, Rakelbeschichtung, Vorhanggiessverfahren, Aufpinseln, Sprühen und Reverse Rollbeschichtung. Es ist auch möglich, die lichtempfindliche Schicht auf einen temporären, flexiblen Träger zu bringen, und dann durch Schichtübertragung via Lamination das endgültige Substrat, z. B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weit variablen Schichtdicken eingesetzt werden können. Dieser Schichtdickenbereich umfasst Werte von ca. 0,5 μm bis mehr als 100 μm. Mit konventionellen Positiv-Systemen auf Naphthochinondiazid-Basis sind Schichtdicken von grösser als 10 μm nicht mehr verwendbar.

Mögliche Einsatzgebiete der erfindungsgemässen Zusammensetzungen sind die Verwendung als Photoresists für die Elektronik (Galvanoresist, Aetzresist, Lötstoppresist), die Herstellung von Druckplatten, wie Offsetdruckplatten oder Siebdruckformen, der Einsatz beim Formteilaetzen oder der Einsatz als Mikroresist bei der Herstellung integrierter Schaltkreise. Dementsprechend unterschiedlich sind die möglichen Schichtträger und die Verarbeitungsbedingungen der beschichteten Substrate.

Für photographische Informationsaufzeichnung dienen z. B. Folien aus Polyester, Celluloseacetat oder mit Kunststoff beschichtete Papiere ; für Offsetdruckformen speziell behandeltes Aluminium und für die Herstellung gedruckter Schaltungen kupferkaschierte Laminate. Die Schichtdicken für photographische Materialien und Offsetdruckformen betragen ca. 0,5 bis 10 μm ; für gedruckte Schaltungen 1 bis ca. 100 μm.

Nach dem Beschichten wird das Lösungsmittel in der Regel durch Trocknen entfernt und es resultiert eine Schicht des Photoresist auf dem Träger.

Nach der in üblicher Weise erfolgten bildmässigen Belichtung des Materials werden die belichteten Stellen des Photolackes durch Herauslösen in einem Entwickler entfernt.

Die Wahl des jeweiligen Entwicklers richtet sich nach der Art des Photolackes, insbesondere nach der Natur des verwendeten Bindemittels oder der entstehenden Photolyseprodukte. Der Entwickler kann wässrige Lösungen von Basen umfassen, denen gegebenenfalls organische Lösungsmittel oder deren Mischungen zugesetzt wurden. Bevorzugt wird die Verwendung einer wässrigen Lösung einer Base als Entwicklungsmittel. Säuren lassen sich nicht anwenden, da deren Einsatz zur Spaltung des β-Acetalesters bzw. β-Ketalesters (oder des entsprechenden Amids) an den unbelichteten Stellen des beschichteten Substrates führen würde, und somit die Ablösung der gesamten Lackschicht zur Folge hätte.

Besonders bevorzugt als Entwickler werden wässrig alkalische Lösungen, wie sie auch für die Entwicklung von Naphthochinondiazidschichten eingesetzt werden. Dazu zählen insbesondere wässrige

Lösungen von Alkalimetallsilikaten, -phosphaten und -hydroxiden. Diesen Lösungen können gegebenenfalls noch kleinere Mengen an Netzmittel und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Ein typisches wässrig/organisches Entwicklungssystem basiert auf Butylcellosolve/Wasser (maximal 30 Gew.% Wasseranteil).

Der Begriff « Belichtung mit einem vorbestimmten Muster aktinischer Strahlung » beinhaltet sowohl die Belichtung durch eine Photomaske, die ein vorbestimmtes Muster enthält, beispielsweise ein Diapositiv, sowie die Belichtung durch einen Laserstrahl, der beispielsweise computergesteuert über die Oberfläche des beschichteten Substrates bewegt wird, und auf diese Weise ein Bild erzeugt.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich. Als Lichtquellen kommen daher eine grosse Anzahl der verschiedensten Typen zur Anwendung. Es sind sowohl Punktlichtquellen als auch flächenförmige Strahler (Lampenteppiche) geeignet. Beispiele sind : Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen und photographische Flutlichtlampen. Der Abstand zwischen Lampe und erfindungsgemässen Bildmaterial kann je nach Anwendungszweck und Lampentyp bzw. -stärke variieren, z. B. zwischen 2 cm bis 150 cm. Speziell geeignet sind Laserlichtquellen, z. B. Argonionenlaser Kryptonionenlaser mit starken Emissionslinien (Art-Laser) bei 457, 476, 488, 514, 528 nm. Bei dieser Art der Belichtung ist keine Photomaske im Kontakt mit der Photopolymerschicht mehr nötig ; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die lichtempfindlichen Zusammensetzungen können gegebenenfalls auch Sensibilisatoren enthalten, um die spektrale Empfindlichkeit in einem bestimmten Bereich des elektromagnetischen Spektrums zu erhöhen. Zu diesen Sensibilisatoren zählen beispielsweise Michlers Keton, Benzophenone, Thioxanthone oder aromatische Kohlenwasserstoffe wie Anthracen, Pyren oder Perylen.

Das Bestrahlen mit Elektronenstrahlen ist eine weitere Abbildungsmöglichkeit. Elektronenstrahlen können die erfindungsgemässe Zusammensetzung durchgreifend zersetzen und auch gegebenenfalls vernetzen, so dass ein negatives Bild entsteht, wenn die unbestrahlten Teile durch Lösungsmittel oder Belichten ohne Vorlage und Entwickeln entfernt werden. Bei geringer Intensität und/oder höherer Schreibgeschwindigkeit des Elektronenstrahls bewirkt dagegen der Elektronenstrahl eine Differenzierung in Richtung höherer Löslichkeit, d. h., die bestrahlten Schichtpartien können vom Entwickler entfernt werden. Wenn das Verfahren mit Elektronenstrahlen durchgeführt wird, sind neben den bekannten für sichtbares und nahes UV Licht empfindlichen photolytischen Säurespendern auch solche geeignet, deren Absorptionsbereiche im kürzerwelligeren Teil des elektromagnetischen Spektrums liegen und die damit gegenüber Tageslicht wenig empfindlich sind. Dies hat den Vorteil, dass man die Aufzeichnungsmaterialien ohne Lichtausschluss handhaben kann und dass die Materialien besser lagerfähig gemacht werden können. Als Beispiele für derartige Starter sind Tribrommethylphenylsulfon, 2,2'-4,4'-6,6'-Hexabromdiphenylamin, Pentabromethan, 2,3,4,5-Tetrachloranilin, Pentaerithrittetrabromid, Clophen-Harz W, d. h., ein Chlorterphenylharz, oder chlorierte Paraffine zu nennen. Die erfindungsgemässen Zusammensetzungen können in den unterschiedlichsten Einsatzgebieten Verwendung finden. Sie werden insbesondere dort eingesetzt, wo eine photolithographische Abbildung mit hoher Auflösung hergestellt werden soll.

Die Erfindung betrifft daher auch die Verwendung der Zusammensetzungen, wie oben definiert, als Positiv-Photoresists für die Herstellung von Druckformen, gedruckten Schaltungen oder integrierten Schaltkreisen, sowie für silberlose photographische Filme. Ferner betrifft die Erfindung die unter Verwendung besagter Zusammensetzungen hergestellten Druckformen, gedruckten Schaltungen oder integrierten Schaltkreise oder silberlosen photographischen Filme.

Herstellungsbeispiele

Beispiel 1

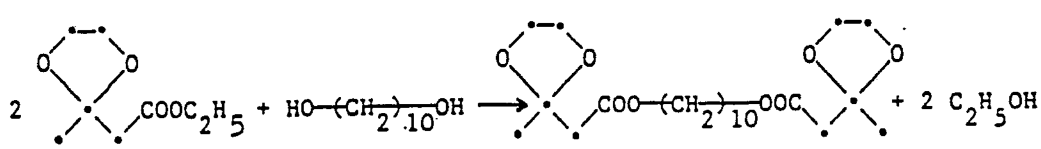

In einem Reaktionskolben, versehen mit Rührer, Destillationsaufsatz und Stickstoffeinleitung erhitzt man die Mischung aus :

522,6 g (3 Mol) Acetessigsäureethylesterethylenacetal
174,3 g (1 Mol) 1,10-Decandiol
7,0 g Kaliumcyanid

auf 140 °C und destilliert den sich bildenden Ethylalkohol unter leichtem Stickstoffstrom während 5 h bei dieser Temperatur ab. Die erkaltete und mit 300 ml Toluol verdünnte Reaktionsmischung wird mit gesättigter wässriger Natriumcarbonatlösung und mit gesättigter Natriumchloridlösung gewaschen, über Kaliumcarbonat getrocknet, und das Lösungsmittel am Rotationsverdampfer abgezogen. Der Rückstand wird 2 h am Hochvakuum bei 150° Badtemperatur gehalten, wobei der Ueberschuss an Acetessigesteracetal abdestilliert (ca. 150 g Destillat). Es verbleiben 409 g (95 % d. Th.) des gewünschten Acetaldiesters als bräunliches Oel.

Elementaranalyse :
berechnet (%) C 61,37 H 8,90
gefunden (%) C 61,12 H 8,96

Dünnschicht :
Silicagel, Laufmittel : Methylenchlorid/Methanol = 95/5 Vol. T. ; Anfärbung mit Kaliumpermanganatlösung : 1 Hauptfleck (Produkt) + 1 schwacher Nebenfleck

[1]N-NMR Spektrum (100 MHZ ; $CDCl_3$) : δ Werte (ppm) 1,5 (s+m, 22H) ; 2,7 (S, 4H) ; 4,0 (s+m, 12H)

Beispiel 2

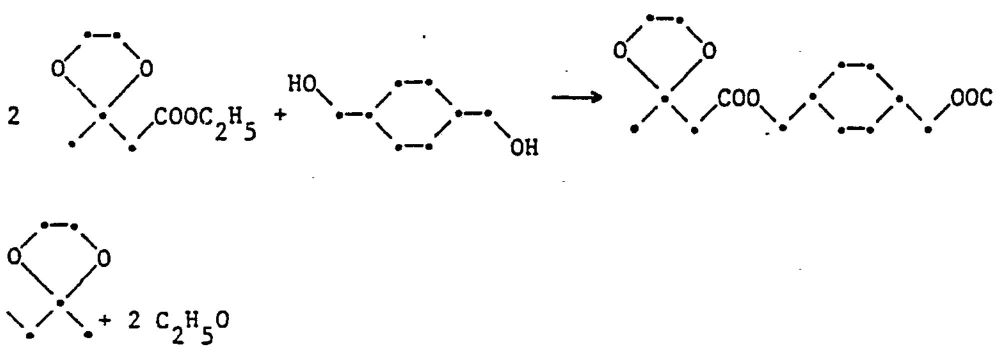

gemäss dem Verfahren von Beispiel 1 aus :
20,9 g (0,12 Mol) Acetessigsäureethylesterethylenacetal
7,2 g (0,04 Mol) 1,4-Bis(hydroxymethyl)cyclohexan cis + trans (80 % trans-Gehalt)
0,28 g Kaliumcyanid
Man gewinnt 11,6 g (72 % d. Th.) des gewünschten Acetal-diesters als bräunliches Oel.

Elementaranalyse :
berechnet (%) C 59,93 H 8,05
gefunden (%) 60,15 8,06

Dünnschicht :
1 Hauptfleck + schwacher Nebenfleck

$^1$H-NMR Spektrum (100 MHZ ; $CDCl_3$) : δ Werte (ppm) 1,5 (s+m, 16 H) ; 2,7 (s, 4 H) ; 4,0 (s+m, 12 H)

Beispiel 3

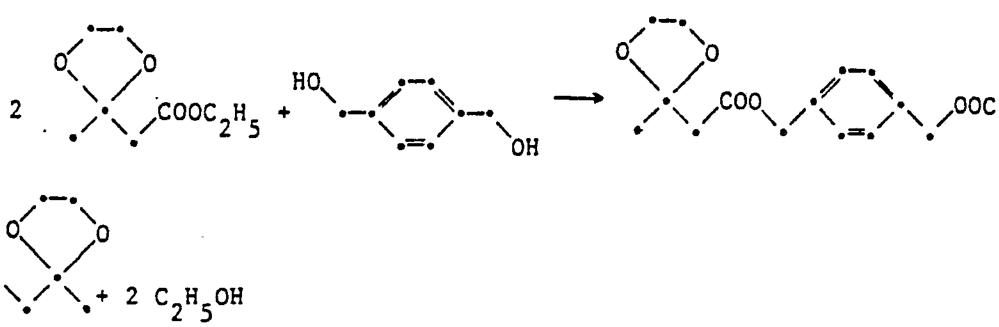

gemäss dem Verfahren von Beispiel 1 aus :
10,45 g (0,06 Mol) Acetessigsäureethylesterethylenacetal
2,76 g (0,02 Mol) 1,4-Benzol-dimethanol
0,13 g Kaliumcyanid
Man gewinnt 6,25 g (79 % d. Th.) des gewünschten Acetal-diesters als gelbliches Oel.

Elementaranalyse :
berechnet (%) C 60,90 H 6,64
gefunden (%) C 61,47 H 6,68

Dünnschicht :
1 Hauptfleck + schwacher Nebenfleck

$^1$H-NMR Spektrum (100 MHz ; $CDCl_3$) : δ Werte (ppm) 1,5 (s, 6 H) ; 2,7 (s, 4 H) ; 4,0 (s, 8 H) ; 5,1 (s, 4 H) ; 7,3 (s, 4 H)

Beispiel 4

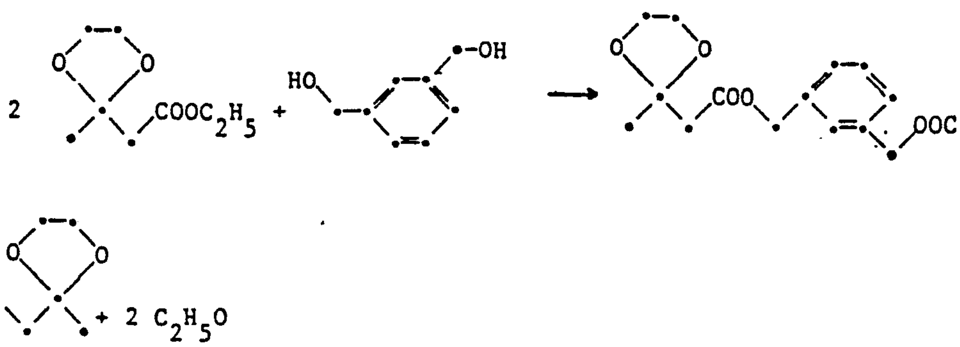

gemäss Verfahren Beispiel 1 aus :
10,45 g (0,06 Mol) Acetessigsäureethylesterethylenacetal
2,76 g (0,02 Mol) 1,3-Benzol-dimethanol
0,13 g Kaliumcyanid
Man erhält 6,15 g des gewünschten Acetal-diesters als gelbliches Oel.

Elementaranalyse :
berechnet (%) C 60,90 H 6,64
gefunden (%) C 61,27 H 6,74

Dünnschicht :
Hauptfleck + schwacher Nebenfleck

$^1$H-NMR Spektrum : (100 MHz ; $CDCl_3$) : δ Werte (ppm) 1,5 (s, 6 H) ; 2,7 (s, 4 H), 4,0 (s, 8 H), 5,1 (s, 4 H) ; 7,3 (m, 4 H)

Beispiel 5

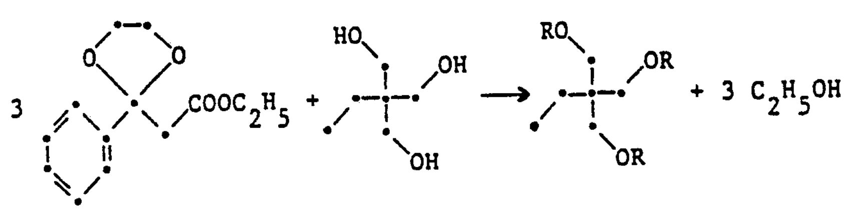

R = 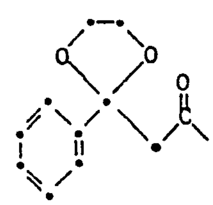

gemäss Verfahren Beispiel 1 aus :

18,90 g (0,08 Mol) Benzoylessigsäureethylester-ethylenketal
2,68 g (0,02 Mol) Trimethylolpropan
0,17 g Kaliumcyanid

Man erhält 10,0 g (71 % d. Th.) des gewünschten Acetal-triesters als bräunliches Harz.

Elementaranalyse :

berechnet (%) C 66,47 H 6,29
gefunden (%) C 67,01 H 6,21

$^1$H-NMR Spektrum (100 MHz ; $CDCl_3$) : δ Werte (ppm) 0,5-2,0 (m,) ; 3,0 (s + m) ; 3,5-5,0 (m) ; 5,9 (s) ; 7,30 (m)

Beispiel 6

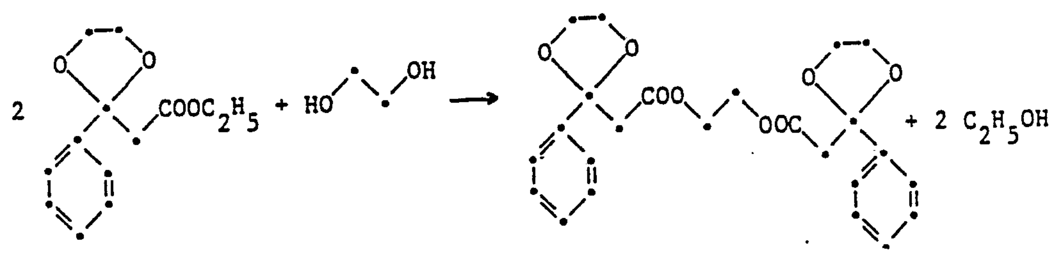

gemäss Verfahren Beispiel 1 aus :

27,35 g (0,12 Mol) Benzoylessigsäureethylester-ethylenketal
3,10 g (0,05 Mol) Ethylenglykol
0,74 g 30 %-ige Lösung von $NaOCH_3$ in $CH_3OH$

Man gewinnt 17,5 g (79 % d. Th.) des gewünschten Acetal-diesters als farbloses Oel.

Elementaranalyse :

berechnet (%) C 65,15 H 5,92
gefunden (%) C 65,32 H 5,88

Dünnschicht :

1 Hauptfleck + 2 schwache Nebenflecken

$^1$H-NMR Spektrum (100 MHz, $CDCl_3$) : d-Werte (ppm) 3,0 (m, 4 H) ; 3,5-4,8 (m, 12 H) ; 7,3 (m, 10 H)

Beispiel 7

a) 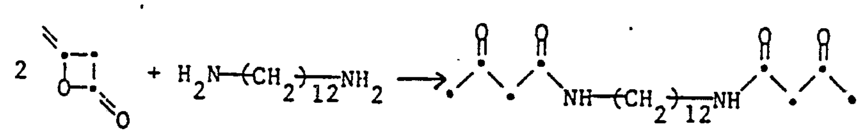

20,0 g (0,10 Mol) 1,12-Diaminododecan in 150 ml Ethylalkohol werden auf 0 °C gekühlt und 18,0 g (0,21 Mol) Diketen tropfenweise innert ca. 1 Stunde unter Kühlung zugegeben. Die weisse Suspension verdünnt man mit 150 ml Ethylalkohol, filtriert und kristallisiert den Rückstand heiss aus 250 ml Ethylalkohol um. Nach dem Trocknen (24 Stunden bei 45 °C im Vakuum) verbleiben 30,0 g (81 % d. Thl.) des Bis-Acetessigsäureamids als farbloses, kristallines Produkt vom Smp. 155-156 °C.

Elementaranalyse :

berechnet (%) C 65,19 H 9,85 N 7,60
gefunden (%) C 65,25 H 9,73 N 7,57

b) 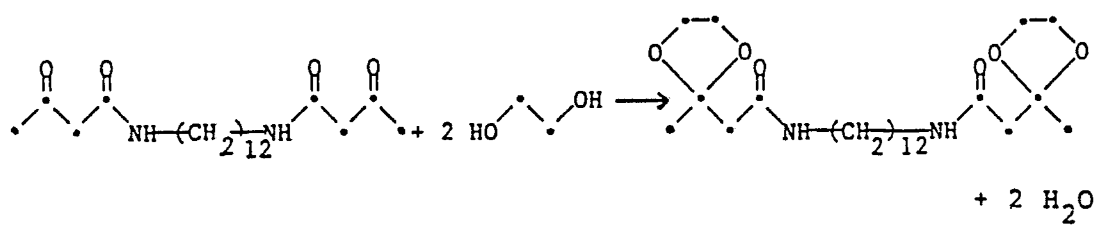

Das Gemisch aus :
29,5 g (0,08 Mol) Bis-acetessigsäureamid aus a)
11,9 g (0,19 Mol) Ethylenglykol
0,16 g p-Toluolsulfonsäure
30 ml Chlorbenzol
15 ml Benzol
wird am Wasserabscheider während 8 Stunden am Rückfluss gehalten (nach 4 Stunden gibt man noch 2 ml Ethylenglykol zu). Man lässt auf Raumtemperatur abkühlen und filtriert die Suspension. Nach dem Trocknen (4 Stunden bei 80° im Vakuum) gewinnt man 34,0 g (93 % d. Th.) des gewünschten Acetaldiamids als farbloses, kristallines Produkt von Smp. 92-103 °C.

Elementaranalyse :

| | | | |
|---|---|---|---|
| berechnet (%) | C 63,13 | H 9,71 | N 6,14 |
| gefunden (%) | C 63,18 | H 9,71 | N 6,03 |

Dünnschicht :
Silicagel, Laufmittel : Methylenchlorid/Methanol = 95/5 Vol. T. ; Anfärbung mit Iod
1 Hauptfleck (Produkt).

$^1$H-NMR Spektrum (100 MHz, $CDCl_3$) :
a) δ-Werte (ppm) 1,1 (m) ; 2,1 (s) ; 2,8-3,5 (m)
b) δ-Werte (ppm) 1,1 (m, 26 H) ; 2,6 (s, 4 H) ; 3,3 (m, 4 H), 4,0 (s, 8 H), 6,5 (s (breit), 2 H)

## Anwendungsbeispiele

Uebersicht über die jeweils eingesetzten Entwickler

Entwickler A :
75,0 g Natrium-metasilicat pentahydrat
0,4 g Supronic® B50 [ABM chemicals Ltd. Stockport Cheshire SK61PQ/GB] ; nichtionisches Netzmittel
925,0 g Deionisiertes Wasser

Entwickler B : Entwickler A + $H_2O$ = 4 + 1 (Vol. T.)

Entwickler C : Entwickler A + $H_2O$ = 1 + 1 (Vol. T.)

## Beispiel I

Beschichtungslösung :

| | |
|---|---|
| Alnovol® PN 430 (Novolak der Fa. Hoechst) 25 %-ig gelöst in Ethylglykolacetat | 8,00 g |
| Acetaldiester aus Beispiel 1 25 %-ig gelöst in Ethylglykolacetat | 2,00 g |
| 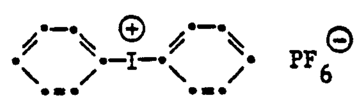 | 0,125 g |
| Farbstoff Orasolrot B (Ciba-Geigy) | 0,0125 g |

Die Lösung wird mittels eines Ziehrakels in ca. 20 μm Nassfilmdicke auf ein anodisiertes und elektrolytisch aufgerauhtes Aluminiumblech (Offsetplattensubstrat) beschichtet und 15 Minuten bei 80° getrocknet. Es resultiert ein Trockenauftrag von 4 g/m².

Man belichtet durch einen in der Offsetplattenkopie üblichen Testkeil, in diesem Falle den UGRA-Keil (Switzerland). Lichtquelle ist eine 5 000 Watt Metall-Halogenlampe (Sylvania M 061) im Abstand von 65 cm zum Vakuumrahmen. Das belichtete Material wird im Entwickler C durch schwaches Reiben mit einem Wattebausch entwickelt.

Resultat :
Belichtungszeit : 60 Sekunden
Entwicklungszeit : 30 Sekunden Entwickler C bei 20 °C
Halbtonkeilstufen : 1-3 ausentwickelt
4-6 angegriffen

Das unbelichtete Material ist auch nach 4 Minuten im Entwickler C nicht angegriffen.

### Beispiele II-VI

Analog zu Beispiel I, aber mit anderen Acetaldiestern, werden Belichtungsversuche durch einen Testkeil durchgeführt.

Resultate :

| Beispiel | II | III | IV | V | VI |
|---|---|---|---|---|---|
| Acetaldiester Herst.beispiel | 2 | 3 | 4 | 5 | 7b |
| Trockenauftrag ($g/m^2$) | 4,3 | 5,5 | 4,6 | 6,9 | 6,0 |
| Belichtungszeit (sek.) | 60 | 60 | 60 | 60 | 60 |
| Entwickler | B | B | B | A | C |
| Entwicklungszeit (sek.) | 30 | 15 | 15 | 300 | 240 |
| Halbtonkeil ausentwickelt | 1-3 | 1-2 | 1-2 | 1 | 1 |
| Halbtonkeil angegriffen | 4-6 | 3-5 | 3-5 | 2-4 | 2-4 |

### Beispiel VII

Dieses Beispiel zeigt eine Beschichtung auf Kupfer, wie sie zur Herstellung einer Leiterplatte gebraucht wird.

Beschichtungslösung :

Verwendet wird eine Beschichtungslösung gemäss Beispiel I. Mittels eines Ziehrakels (20 µm Nassfilmdicke) wird eine gereinigte kupferkaschierte Leiterplatte damit beschichtet. Man trocknet 15 Minuten bei 80°.

In der in Beispiel I beschriebenen Belichtungsapperatur wird durch eine Stauffer Sensitivity guide (Inkremente der opt. Dichte = 0,15) während 60 Sekunden belichtet.

Resultat :

Belichtungszeit : 60 Sekunden
Entwicklungszeit : 60 Sekunden Entwickler C bei 20°
Halbtonkeitstufen : 1-4 ausentwickelt
5-7 angegriffen

Das entwickelte Resistbild lässt sich als Aetzmaske zur Wegätzung des Kupfers in den üblichen Bädern (z. B. $FeCl_3$-Lösung) verwenden.

### Beispiel VIII

Dieses Beispiel zeigt die Herstellung und Verarbeitung einer dicken Schicht (50 µm) auf Kupfer.

Beschichtungslösung :

| | |
|---|---|
| Alnovol® PN 430, 50 %-ige Lösung in Ethylglykolacetat | 4,00 g |
| Acetaldiester aus Beispiel 1, 50 %-ige Lösung in Ethylglykolacetat | 1,00 g |
| 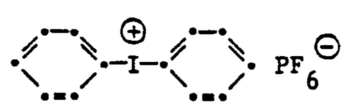 | 0,125 g |
| Farbstoff Orasolrot B | 0,0125 g |

Die Lösung wird gemäss Beispiel I mit einem Ziehrakel, Nassfilmdicke 100 µm, auf eine kupferkaschierte Leiterplatte beschichtet. Man trocknet 30 Minuten bei 100 °C. Die Dicke der lichtempfindlichen Schicht beträgt ca. 50 µm. Zur Belichtung wird die beschriebene Lichtquelle (Beispiel I) verwendet. Als Vorlage dient der beschriebene (Beispiel VII) Stauffer-Testkeil sowie ein Leiterbahnenmuster.

Resultat :

Belichtungszeit : 60 Sekunden
Entwicklungszeit : 4 Minuten Entwickler C bei 20°
Halbtonkeitstufen : 1-5 ausentwickelt
6-7 angegriffen

Das entwickelte Resistbild kann z. B. als Galvanoresist für die Semi-Additiv-Technik dienen.
Bemerkenswert ist die kurze Belichtungszeit von 60 Sekunden bei einer Schichtdicke von 50 µm.

Beispiel IX

Dieses Beispiel wird analog zu Beispiel I ausgeführt. Dabei wird aber 2-(4-Methoxystyryl)-4,6-bis-trichlormethyl-s-triazin (Beispiel 1 aus DOS 2,243,621) als Säurespalter anstelle von Diphenyliodoniumhexafluorophosphat verwendet.
Lichtquelle ist eine 2 000-Watt Metall-Halogenlampe im Abstand von 75 cm.

Resultat :
Acetaldiester : gemäss Beispiel I
Trockenauftrag : 4,6 g/$m^2$
Belichtungszeit : 60 Sekunden
Entwickler : Typ A
Entwicklungszeit : 180 Sekunden
Halbtonkeil : 1-3 ausentwickelt
4-5 angegriffen

**Patentansprüche**

1. Verbindungen der Formel I

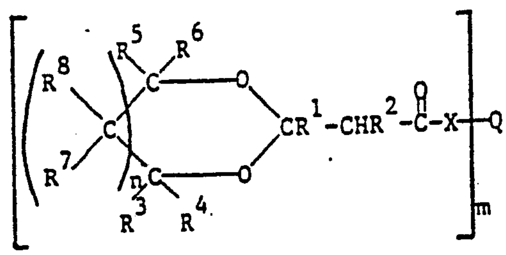

(I)

worin $R^1$ und $R^2$ unabhängig voneinander Wasserstoff, $C_1$-$C_8$ Alkyl, Phenyl, Naphthyl, $C_5$-$C_7$ Cycloalkyl, $C_7$-$C_{14}$ Aralkyl oder $C_7$-$C_{14}$ Alkaryl bedeuten, wobei besagte organische Reste gegebenenfalls durch ein bis drei Halogenatome, Hydroxy-, Alkoxy-, Nitro-, Cyan- oder Aminogruppen substituiert sind, worin $R^3$, $R^4$, $R^5$, $R^6$, $R^7$ und $R^8$ unabhängig voneinander Wasserstoff oder $C_1$-$C_4$ Alkyl bedeuten, worin X —O— oder —$NR^9$— ist, worin $R^9$ Wasserstoff oder $C_1$-$C_4$ Alkyl ist, n 0 oder 1 bedeutet, m 2, 3 oder 4 ist, und worin Q einen m-wertigen aliphatischen, cycloaliphatischen, aromatischen, araliphatischen oder fünf- oder sechsgliedrigen heterocyclischen Rest darstellt, wobei beim Vorliegen mehrerer Ester- oder Amidreste im Molekül, die Gruppen $R^1$ bis $R^9$ innerhalb der oben gegebenen Definitionen unterschiedlich sein können, und wobei im Falle n = 0 die beiden Kohlenstoffatome direkt mit einander verbunden sind, so dass ein fünfgliedriger heterocyclischer Rest entsteht.

2. Verbindungen der Formel I gemäss Anspruch 1, worin $R^1$ und $R^2$ Wasserstoff, $C_1$-$C_4$ Alkyl oder Phenyl sind, $R^3$, $R^4$, $R^5$ und $R^6$ unabhängig voneinander Wasserstoff oder Methyl bedeuten, X —O— oder —NH— darstellt, n 0 bedeutet, m 2 oder 3 ist, und worin Q ein m-wertiger aliphatischer, cycloaliphatischer, aromatischer oder araliphatischer Rest ist.

3. Verbindungen der Formel I gemäss Anspruch 1, worin $R^1$ und $R^2$ unabhängig voneinander Wasserstoff, Methyl oder Phenyl sind, $R^3$, $R^4$, $R^5$ und $R^6$ Wasserstoff bedeuten, X —O— oder —NH— ist, n 0 darstellt, m 2 ist, und worin Q ein zweiwertiger Rest ist, der ausgewählt wird aus der Gruppe bestehend aus $C_2$-$C_{18}$ Alkylen, Xylylen oder Hexahydroxylylen.

4. Verbindungen der Formel I gemäss Anspruch 1, worin $R^1$ und $R^2$ unabhängig voneinander Wasserstoff, Methyl oder Phenyl bedeuten, $R^3$, $R^4$, $R^5$ und $R^6$ Wasserstoff darstellen, X —O— oder —NH— ist, n 0 bedeutet, m 3 ist, und worin Q sich ableitet von Trimethylolpropan oder Glycerin nach Entfernung der Alkoholgruppen.

5. Verbindungen der Formel I gemäss Anspruch 1, worin $R^1$ und $R^2$ unabhängig voneinander Wasserstoff, Methyl oder Phenyl bedeuten, $R^3$, $R^4$, $R^5$ und $R^6$ Wasserstoff darstellen, X —O— oder —NH— ist, n 0 ist, m 4 bedeutet, und worin Q sich ableitet von Pentaerithrit nach Entfernung der Alkoholgruppen.

6. Verbindungen der Formel I gemäss Anspruch 1, worin n 0 bedeutet.

7. Verbindungen der Formel I gemäss Anspruch 1, worin m 2 oder 3 ist.

8. Verbindungen der Formel I gemäss Anspruch 1, worin $R^1$ Methyl ist und die Reste $R^2$ bis $R^8$ Wasserstoff darstellen.

9. Verbindungen der Formel I gemäss Anspruch 1, worin X —O— ist.

10. Zusammensetzung enthaltend
a) eine Verbindung der Formel I gemäss Anspruch 1 und
b) eine unter Einwirkung von aktinischer Strahlung eine Säure abspaltende Verbindung.
11. Zusammensetzung gemäss Anspruch 10 enthaltend als zusätzliche Komponente c) ein Bindemittel.
12. Verfahren zur Erzeugung von positiven Abbildungen umfassend folgende Arbeitsschritte :
a) Beschichtung eines Substrates mit einer strahlungsempfindlichen Zusammensetzung gemäss den Ansprüchen 10 oder 11,
b) Belichtung des beschichteten Substrates mit einem vorbestimmten Muster aktinischer Strahlung, und
c) Entwicklung des belichteten Substrates.
13. Verwendung der Zusammensetzung gemäss Ansprüchen 10 oder 11, als Positiv-Resists für die Herstellung von Druckformen, gedruckten Schaltungen oder integrierten Schaltkreisen, sowie für silberlose photographische Filme.
14. Die unter Verwendung der Zusammensetzung gemäss Anspruch 10 hergestellten Druckformen, gedruckten Schaltungen, integrierten Schaltkreise oder silberlosen photographischen Filme.

**Claims**

1. A compound of formula I

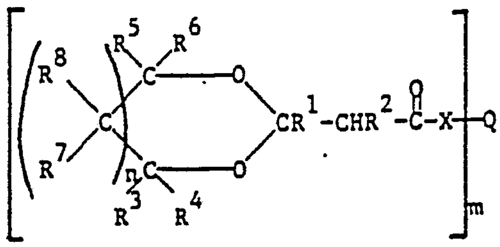

(I)

wherein $R^1$ and $R^2$ are independently of one another hydrogen, $C_1$-$C_8$ alkyl, phenyl, naphthyl, $C_5$-$C_7$ cycloalkyl, $C_7$-$C_{14}$ aralkyl or $C_7$-$C_{14}$ alkaryl, said organic radicals being unsubstituted or substituted by one to three halogen atoms, hydroxy, alkoxy, nitro, cyano or amino groups, wherein $R^3$, $R^4$, $R^5$, $R^6$, $R^7$ and $R^8$ are independently of one another hydrogen or $C_1$-$C_4$ alkyl, wherein X is —O— or —$NR^9$—, wherein $R^9$ is hydrogen or $C_1$-$C_4$ alkyl, n is 0 or 1, m is 2, 3 or 4, and wherein Q is an m-valent aliphatic, cycloaliphatic, aromatic, araliphatic or 5- or 6-membered heterocyclic radical where, if several ester or amide radicals are present in the molecule, the groups $R^1$ to $R^9$ within the scope of the above definitions may be different and, where, if n is 0, both carbon atoms are linked directly to each other to form a 5-membered heterocyclic radical.
2. A compound of formula I according to claim 1, wherein $R^1$ and $R^2$ are hydrogen, $C_1$-$C_4$ alkyl or phenyl, $R^3$, $R^4$, $R^5$ and $R^6$ are independently of one another hydrogen or methyl, X is —O— or —NH—, n is 0, m is 2 or 3, and wherein Q is an m-valent aliphatic, cycloaliphatic, aromatic or araliphatic radical.
3. A compound of formula I according to claim 1, wherein $R^1$ and $R^2$ are independently of one another hydrogen, methyl or phenyl, $R^3$, $R^4$, $R^5$ and $R^6$ are hydrogen, X is —O— or —NH—, n is 0, m is 2, and wherein Q is a divalent radical which is selected from the group consisting of $C_2$-$C_{18}$-alkylene, xylylene or hexahydroxylylene.
4. A compound of formula I according to claim 1, wherein $R^1$ and $R^2$ are independently of one another hydrogen, methyl or phenyl, $R^3$, $R^4$, $R^5$ and $R^6$ are hydrogen, X is —O— or —NH—, n is 0 and m is 3, and wherein Q is derived from trimethylolpropane or glycerol after removal of the alcohol groups.
5. A compound of formula I according to claim 1, wherein $R^1$ and $R^2$ are independently of one another hydrogen, methyl or phenyl, $R^3$, $R^4$, $R^5$ and $R^6$ are hydrogen X is —O— or —NH—, n is 0 and m is 4, and wherein Q is derived from pentaerythritol after removal of the alcohol groups.
6. A compound of formula I according to claim 1, wherein n is 0.
7. A compound of formula I according to claim 1, wherein m is 2 or 3.
8. A compound of formula I according to claim 1, wherein $R^1$ is methyl and the radicals $R^2$ to $R^8$ are hydrogen.
9. A compound of formula I according to claim 1, wherein X is —O—.
10. A composition containing
a) a compound of formula I according to claim 1 and
b) a compound that releases acid under the action of actinic radiation.
11. A composition according to claim 10, containing a binder as additional component c).
12. A process for producing positive images comprising the following steps :
a) coating a substrate with a radiation-sensitive composition according to claims 10 or 11,

b) exposing the coated substrate to actinic radiation having a predetermined pattern, and

c) developing the exposed substrate.

13. Use of the composition according to claims 10 or 11 as a positive resist for the production of printing plates, printed circuits or integrated circuits and also for silver-free photographic films.

14. The printing plate, printed circuits, integrated circuits or silver-free photographic films produced using the composition according to claim 10.

**Revendications**

1. Composés de formule I :

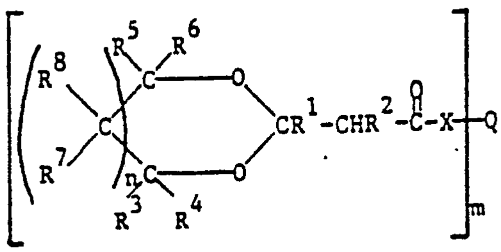

(I)

dans laquelle $R^1$ et $R^2$ désignent chacun, indépendamment l'un de l'autre, l'hydrogène ou un groupe alkyle en $C_1$-$C_8$, phényle, naphtyle, cycloalkyle en $C_5$-$C_7$, aralkyle en $C_7$-$C_{14}$ ou alcaryle en $C_7$-$C_{14}$, groupes qui peuvent éventuellement porter de 1 à 3 atomes d'halogènes ou groupes hydroxy, alcoxy, nitro, cyano ou amino, $R^3$, $R^4$, $R^5$, $R^6$, $R^7$ et $R^8$ désignent chacun, indépendamment les uns des autres, l'hydrogène ou un alkyle en $C_1$-$C_4$, X représente l'oxygène ou un groupe —$NR^9$—, $R^9$ étant l'hydrogène ou un alkyle en $C_1$-$C_4$, n est le nombre 0 ou 1, m le nombre 2, 3 ou 4 et Q un radical de valence m, aliphatique, cycloaliphatique, aromatique, araliphatique ou hétérocyclique à 5 ou 6 chaînons, dans le cas où la molécule comporte plusieurs radicaux esters ou amides les groupes $R^1$ à $R^9$ pouvant être différents les uns des autres dans les limites des définitions que l'on vient d'indiquer, et si n = 0 les deux atomes de carbone sont directement liés l'un à l'autre en formant un radical hétérocyclique pentagonal.

2. Composés de formule I selon la revendication 1 dans lesquels $R^1$ et $R^2$ sont chacun l'hydrogène, un alkyle en $C_1$-$C_4$ ou un phényle, $R^3$, $R^4$, $R^5$ et $R^6$ sont chacun, indépendamment les uns des autres, l'hydrogène ou un méthyle, X est l'oxygène ou le groupe —NH—, n = 0, m = 2 ou 3 et Q est un radical de valence m, aliphatique, cycloaliphatique, aromatique ou araliphatique.

3. Composés de formule I selon la revendication 1 dans lesquels $R^1$ et $R^2$ sont chacun l'hydrogène, un méthyle ou un phényle, $R^3$, $R^4$, $R^5$ et $R^6$ sont chacun l'hydrogène, X est l'oxygène ou le groupe —NH—, n = 0, m = 2 et Q est un radical divalent choisi parmi des alkylènes en $C_2$-$C_{18}$ et les groupes xylylènes et hexahydroxylylènes.

4. Composés de formule I selon la revendication 1 dans lesquels $R^1$ et $R^2$ sont chacun l'hydrogène, un méthyle ou un phényle, $R^3$, $R^4$, $R^5$ et $R^6$ sont chacun l'hydrogène, X est l'oxygène ou le groupe —NH—, n = 0, m = 3 et Q provient du triméthylol-propane ou du glycérol par élimination des groupes alcooliques.

5. Composés de formule I selon la revendication 1 dans lesquels $R^1$ et $R^2$ sont chacun l'hydrogène, un méthyle ou un phényle, $R^3$, $R^4$, $R^5$ et $R^6$ sont chacun l'hydrogène, X est l'oxygène ou le groupe —NH—, n = 0, m = 4 et Q provient du pentaérithritol par élimination des groupes alcooliques.

6. Composés de formule I selon la revendication 1 dans lesquels n = 0.

7. Composés de formule I selon la revendication 1 dans lesquels m = 2 ou 3.

8. Composés de formule I selon la revendication 1 dans lesquels $R^1$ est le groupe méthyle et $R^2$ à $R^8$ sont l'hydrogène.

9. Composés de formule I selon la revendication 1 dans lesquels X est l'oxygène.

10. Composition qui comprend :

a) un composé de formule I selon la revendication 1 et

b) un composé libérant un acide sous l'action d'un rayonnement actinique.

11. Composition selon la revendication 10 qui contient en outre un liant (c).

12. Procédé de production d'images positives qui comprend les opérations suivantes :

a) revêtement d'un substrat d'une composition sensible aux rayonnements selon la revendication 10 ou 11,

b) éclairage du substrat recouvert par un rayonnement actinique de modèle déterminé, et

c) développement du substrat éclairé.

13. Utilisation d'une composition selon la revendication 10 ou 11 comme réserve positive pour la fabrication de formes d'impression, circuits imprimés et circuits intégrés, ainsi que pour des pellicules photographiques non argentiques.

14. Les formes d'impression, circuits imprimés et circuits intégrés, ainsi que pellicules photographiques non argentiques, qui ont été obtenus avec la composition de la revendication 10 ou 11.